# EUROPEAN PATENT APPLICATION

(11) **EP 1 113 072 A1**
(43) Date of publication of application: **04.07.2001**
(21) Application number: 99204568.2
(22) Date of filing: 28.12.1999
(51) Int. Cl.: C12N 5/06, C12N 5/08, A61L 27/38

(54) **Tissue engineering using mandibular cells**

(71) Applicant: IsoTis B.V., 3723 MB Bilthoven (NL)
(72) Inventor: The designation of the inventor has not yet been filed
(74) Representative: Ottevangers, Sietse Ulbe

(57) **Abstract**

The invention relates to the field of tissue engineering. The invention particularly relates to tissue engineering implants for reconstructive surgery of bone tissue in the maxillo-facial area.

## Description

The invention relates to the field of tissue engineering. The invention particularly relates to tissue engineering implants for reconstructive surgery of bone tissue in the maxillo-facial area.

To reconstruct extensive defects in the oro-maxillofacial area, harvested autogeneous bone from the iliac crest is usually used. However, based on embryological similarity, i.e. membranous bone of the cranium versus enchondral bone of the iliac crest, the ectomesenchymal mandibular symphysis graft (chin bone) could result in higher success rates.

It has been proposed to harvest mandibular bone tissue from bone plugs, and to use this tissue in the repair of defects in, particularly the maxillo-facial area. A drawback, however, is that only small amounts of bone can be harvested from the chin region. This is the reason why in practice surgeons still tend to make use of bone harvested from other areas.

The present invention seeks to overcome the problems that are currently associated with the use of mandibular bone as a source for tissue that can be used in reconstructive surgery.

Surprisingly, it has been found that sufficient cells can be harvested from a biopsy taken from the chin region of a patient to allow for a feasible tissue engineering protocol. Accordingly, the invention relates to the use of mandibular cells for tissue engineering.

It is advantageous, particularly for applications in reconstructive surgery in the maxillo-facial area, that bone which is obtained by tissue engineering using mandibular cells has the same embryological origin as the tissue surrounding the place of implantation. Mandibular bone tissue has more cortical bone when compared to enchondral bone, from the iliac crest, and has thus stronger mechanical properties. Tissue engineered bone that has been obtained in accordance with the invention has characteristics which more closely resemble those of the surrounding bone in the maxillo-facial area, when compared to tissue engineered bone that has been obtained using cells from the iliac crest.

Furthermore, it is advantageous that a biopsy in the mandibular region is much less painful and discomforting for a patient than a biopsy from the iliac crest. Moreover, hardly any scar tissue will develop from such a biopsy, if at all.

In the context of the present invention, the term tissue engineering refers to a process wherein cells are seeded onto a scaffold and subsequently cultured to form a desired type of tissue. This may result in a medical implant comprising a substrate formed by the scaffold and tissue. In accordance with the invention, the tissue that is engineered is preferably (human) bone tissue.

A matrix to be used as a scaffold in accordance with the invention preferably is biodegradable and biocompatible. It is to be noted, however, that for some applications, e.g. in the field of dental restorations, it may be preferred that the scaffold is substantially non-biodegradable.

In the context of the present invention, the term biocompatible is intended to refer to materials which may be incorporated into a human or animal body substantially without unacceptable responses of the human or animal. The term biodegradable refers to materials which, after a certain period of time, are broken down in a biological environment. Preferably, the rate of breakdown is chosen similar or identical to the rate at which the body generates autogenous tissue to replace the implant of which the biodegradable material is manufactured.

Further, the present matrix is preferably porous or fibrous. It is preferred that the pores in the present matrix are interconnected. For some purposes, for instance in case the tissue engineered product is to be used as a bone filler, it is preferred that the product is porous substantially throughout its structure. In other cases, such as for use as an implant, it may be desired that the porosity is primarily or substantially only present at and near the surface of the product.

Preferably, the matrix has a macroporosity between 30 and 99%, more preferably between 60 and 95%. The pores in the matrix preferably have a diameter of between 0.1 and 2000 µm, more preferably between 50 and 1000 µm. The macroporosity and the diameter of the pores will be chosen such that, on the one hand, sufficient diffusion of nutrients and waste products can take place, and, on the other hand, sufficient mechanical strength is provided by the matrix.

Preferred materials to be used to form the matrix are ceramic and glass materials. Suitable glass materials are Bioglasses or glass-ceramics. Examples of suitable ceramic materials include calcium phosphate, calcium carbonates, and sodium calcium phosphates. Particularly suitable ceramic materials are chosen from the group of octacalcium phosphate, apatites, such as hydroxyapatite and carbonate apatite, whitlockites, such as α-tricalcium phosphate, β-tricalcium phosphate, sodium calcium phosphate, and combinations thereof. The use of ceramic materials has been found to lead to particular good attachment of cells both in vivo and in vitro. Also, a ceramic structure may mimic bone in case of a large, full thickness defect.

Other materials that can be used to form the matrix are metals. Particularly for dental applications metals are preferred. Examples of suitable metals include titanium, tantalum, zirconium, niobium, and alloys such as Ti6Al4V.

Another group of preferred materials to be used to form the matrix is the group of polymeric materials, preferably elastomeric materials. Suitable polymers may be of natural or synthetic origin. Particularly preferred polymeric materials are materials having hydrogel properties. These materials allow for diffusion of nutrients, gases and waste to and from cells which may be seeded onto the scaffold. Furthermore, the swelling behavior of these materials allows optimal fixation of the structure in a defect when it is implanted without cells seeded thereto in vitro. In case a polymeric material is used, a porous structure may be obtained by any known method, such as salt leaching or sintering.

Suitable examples of polymeric materials include starch, starch based polymers, collagen, cellulose, cellulose derivatives, polyanhydrides, polyorthoesters, polyglycolic acids, and polylactides. A specific class of polymeric materials, having hydrogel properties and leading to particularly good results is the class of copolymers of a polyalkylene glycol and an aromatic polyester. Preferably, these copolymers comprise 40-80 wt.%, more preferably 50-70 wt.% of the polyalkylene glycol, and 60-20 wt.%, more preferably 50-30 wt.% of the aromatic polyester. A preferred type of copolymers according to the invention is formed by the group of block copolymers.

Preferably, the polyalkylene glycol has a weight average molecular weight of from 150 to 4000, more preferably of 200 to 1500. The aromatic polyester preferably has a weight average molecular weight of from 200 to 5000, more preferably of from 250 to 4000. The weight average molecular weight of the copolymer preferably lies between 20,000 and 200,000, more preferably between 50,000 and 120,000. The weight average molecular weight may suitably be determined by gel permeation chromatography (GPC). This technique, which is known per se, may for instance be performed using tetrahydrofuran as a solvent and polystyrene as external standard. Preferred polyalkylene glycols are chosen from the group of polyethylene glycol, polypropylene glycol, and polybutylene glycol and copolymers thereof, such as poloxamers. A highly preferred polyalkylene glycol is polyethylene glycol.

In a preferred embodiment, the polyester is chosen from the group of polyethylene terephtalate, polypropylene terephtalate, and polybutylene terephtalate. A highly preferred polyester is polybutylene terephtalate.

In a preferred embodiment, the scaffold is provided with a ceramic coating. Preferably, the ceramic coating is a calcium phosphate coating. It has been found that the presence of a ceramic coating is highly beneficial to the attachment of cells to the scaffold.

The calcium phosphate may be applied as disclosed in European patent application 98203085.0 by soaking the scaffold into a highly concentrated calcifying solution at low temperature. The calcifying solution is preferably composed of at least calcium and phosphate ions, and optionally of magnesium, carbonate, sodium and chloride ions, which are dissolved into water by bubbling carbon dioxide gas. During the natural release of carbon dioxide gas or its exchange with air, the pH of the calcifying solution is increased and the saturation is raised until the nucleation of carbonated calcium phosphate crystals on the surface of the artificial skin. The process of bubbling and/or releasing CO₂ gas through or from the calcifying solution can be repeated until a sufficient thickness of the coating has been reached.

In general, the thickness of the ceramic layer will be between 0.1 and 100, preferably between 10 and 40 µm. It is preferred, that the ceramic coating is designed such that it has its beneficial effect during the seeding of cells onto the scaffold, and during the subsequent in vitro culturing of said cells.

The scaffold may be processed to have a particular desired form in any known manner. Particularly when the matrix is formed of a polymeric material having hydrogel properties, the swelling behavior or the scaffold allows for swell fixation of the implant in a defect into which it is implanted. The extent of swelling can suitably be controlled by adjusting the composition of the polymeric material.

The scaffold is to be seeded with cells prior to its implantation. As has been mentioned above, it is an important aspect of the invention that mandibular cells are used. It is preferred that the cells are autologous cells, thus minimizing, or even excluding, the chance of rejection responses in the patient treated with the present scaffold. In the context of the invention, the term mandibular cells refers to living bone cells that are harvested from bone tissue that has been obtained by taking a biopsy from the mandibula, i.e. the lower jawbone.

Thus, the cells used in accordance with the invention may be obtained by harvesting a bone plug from the mandible of a patient. The cells may be collected in a suitable culture medium, to which heparin is preferably added in order to prevent clotting. The cells in the bone plugs may subsequently be detached e.g. by means of squeezing and/or re-suspending the bone plug in a syringe with a needle attached, or by means of enzymatic treatment (e.g. collagenase treatment).

The obtained cells may be derived from either the bone marrow, the cortical bone, trabecular bone or the periost of the bone plug. The cells are preferably culture expanded in a suitable medium (e.g. Dulbecco's Modified Minimal Eagle's Medium (DMEM) or Alpha Minimal Essential Medium (α-MEM)), which preferably comprises one or more growth factors (e.g. basic fibroblastic growth factor (bFGF) or transforming growth factor (TGF) and serum that is preferably synthetic or autologous, but could also be animal derived (e.g. from bovine or horse). In a preferred embodiment, the culture medium comprises at least 300 mg/l of L-glutamine. It is further preferred that the medium comprises 0.1-10 *µ*g/l bFGF, 75-1500 mg/l ascorbic acid and antibiotics, preferably streptomycin and penicillin G. These cultured cells will generally be a mixture of differentiated osteoblastic cells and cells that are not completely differentiated and which are still capable to differentiate into mature osteoblastic cells. The period required for culturing may vary broadly and range between one hour and several months, depending on the seeding concentration on the implants and the size of the implants.

When a desired amount of cultured cells is obtained, the cells may be seeded on a scaffold. The seeding may be carried out in any known manner, for instance by static seeding. It is preferred, however, that the cells are seeded dynamically as has been descriped in co-pending European patent application 98203774.9, which is incorporated herein by reference. The seeded cells may be cultured in a medium that preferably comprises agents that induce differentiation into osteoblasts (e.g. bone morphogenetic proteins or dexamethasone) and/or induce excretion of extracellular matrix and mineralization of said matrix (e.g. β-glycerophosphate or L-ascorbic acid-2-phosphate). The thus obtained hybrid structures may subsequently be used for implantation after an additional culture period that ranges between a few minutes and several weeks.

The invention further relates to the use of the above scaffold as a medical implant in bone tissue repair, particularly in the maxillo-facial area.

The invention will now be elucidated by the following, non-restrictive example.

### EXAMPLE

### MATERIALS AND METHODS

Bone plugs of approximately 0.5ml were harvested from the mandible of 2 female patients of respectively 17 and 32 years old. The bone plugs were collected in 20ml culture medium, to which 1% 5,000 I.E./ml heparin solution was added. The bone plugs were put in a 50ml syringe with a 20G needle attached and re-suspended until most cells had detached from the bone plug. After centrifugation the cells were re-suspended in a culture medium, which included alpha-Minimal Essential Medium, 10% foetal bovine serum, 0.2mM L-ascorbic acid-2-phosphate, 1 ng/L of basic fibroblast growth factor (bFGF) and antibiotics. Third passage cells of both patients were seeded at a density of 2.5x10⁵ to 5x10⁵ cells in porous CaP (calcium phosphate) particles (approximately 2x2x3 mm, mean pore size of 435 micrometer). In addition, 2.5x10⁵ third passage cells of the 17 years old patient were seeded on nonporous titanium discs (Ø=5mm) with a CaP coating of approximately 30µm thick. After a culture period of 1 week in the presence of dexamethasone, twelve tissue/material hybrids were processed for light microscopy, scanning electron microscopy and RT-PCR. With regard to RT-PCR, the cells were immediately examined or after a culture period of respectively 4 and 7 days in the presence and absence of dexamethasone. The expression of markers of the osteoblast phenotype (alkaline phosphatase, osteopontin, osteocalcin, PTH-receptor, type I and III procollagen) as well as a marker for the adipocyte phenotype (lipoprotein lipase) were semi-quantitatively determined. In order to examine the osteoinductive capacity of the tissue/material hybrids, six constructs per condition were subcutaneously implanted in athymic mice for 6 weeks. Furthermore, porous CaP particles and CaP-coated discs without cultured cells were examined before incubation in culture medium, after 1 week incubation in culture medium and after 6 weeks implantation respectively.

### RESULTS

During culture expansion, the cells of both patients exhibited a spindle-shaped fibroblastic morphology. After seeding and culturing the cells for one week on the porous CaP particles and the CaP-coated discs, the substrates were fully covered with a layer of cultured cells. Furthermore, the cells had excreted an extracellular matrix that was visible between the cultured cells. RT-PCR (Reverse-Transcription Polymerase Chain Reaction) indicated that the osteogenic markers exhibited an increased expression during the 7 days of culture both in the presence and absence of dexamethasone. Besides alkaline phosphatase, the expression of the osteogenic markers was not higher in the samples that were cultured in the presence of dexamethasone compared to the samples that were cultured in the absence of dexamethasone.

The implanted samples did not show any signs of inflammation after 6 weeks of subcutaneous implantation. Fibrous tissue and blood vessel ingrowth in the porous particles as well as encapsulation of all hybrid constructs could be detected. Clear de novo bone formation could be detected in the porous CaP particles with cultured tissue. The layer of newly formed bone was in direct contact with the CaP surface of the particles and exhibited many osteocytes and a seam of osteoblasts. In addition, the results obtained with the porous CaP particles and the CaP coated discs in the absence of cultured cells revealed that the particles as well as the CaP coating had remained largely intact during both the in vitro and in vivo period.

### DISCUSSION

The results of this study show that cultured cells obtained from manbibular bone plugs and their excreted extracellular matrix are capable of initiating bone formation in vivo. The presence of the extracellular matrix is expected to improve the bone inducing capacity due to the different growth factors that are present in this matrix. The RT-PCR results confirm the osteogenic capacity of the cultured cells, since all examined osteogenic genes came to expression. The fact that the amount of expression of most osteogenic genes was the same in the cultures that were grown in the presence and absence of dexamethasone indicates that the cultured cells were not stem cells. However, the cells were not fully differentiated into the osteogenic lineage since the amount of expression increased in time.

## Claims

1. Use of mandibular cells for tissue engineering bone.

2. Process for tissue engineering bone, wherein mandibular cells are seeded onto a scaffold and cultured to form bone tissue.

3. Process according to claim 2, wherein the scaffold is of a ceramic, bioglass, metallic or polymeric material.

4. Process according to claim 3, wherein the ceramic material is chosen from the group of octacalcium phosphate, apatites, such as hydroxyapatite and carbonate apatite, whitlockites, such as α-tricalcium phosphate, β-tricalcium phosphate, sodium calcium phosphate, and combinations thereof.

5. Process according to claim 3, wherein the polymeric material is a copolymer of a polyalkylene glycol and an aromatic ester.

6. Process according to claims 2-5, wherein the scaffold is biodegradable and biocompatible.

7. Process according to claims 2-6, wherein the scaffold is provided with a ceramic coating.

8. Process according to claims 2-7, wherein the cells are seeded under static or dynamic conditions.

9. Medical implant comprising a scaffold and bone tissue obtainable by a process according to any of the claims 2-8.

10. Use of a medical implant according to claim 9 for use in reconstructive surgery in the maxillo-facial area.
